# EUROPEAN PATENT APPLICATION

(11) **EP 4 607 532 A1**
(43) Date of publication of application: **27.08.2025**
(21) Application number: 24158978.7
(22) Date of filing: 21.02.2024
(51) Int. Cl.: G16H 40/63

(54) **APPARATUS FOR EXTRACTING INFORMATION ON IMAGE, LAB AUTOMATION AND IMAGE ANALYSIS WORKFLOWS FROM NATURAL LANGUAGE TO AUTOMATE AND ENHANCE REPRODUCIBILITY OF LIFE SCIENCE EXPERIMENTS**

(71) Applicant: Leica Microsystems CMS GmbH, 35578 Wetzlar (DE)
(72) Inventor: KAPPEL, Constantin, 35578 Wetzlar (DE)
(74) Representative: 2SPL Patentanwälte PartG mbB

(57) **Abstract**

A method of generating an instrument setting comprises providing a scientific publication as an input, using a pre-trained language model on the input to extract embeddings of keys and corresponding values associated to settings of a medical instrument. The method further comprises generating an instrument setting based on the embeddings and the corresponding values, the instrument setting causing the medical instrument to perform a task.

## Description

### Background

Reproducibility of an experiment is important in scientific research, which ensures a reliability of data acquired and analyzed using published acquisition parameters and analysis methods. For example, in the biomedical imaging field, currently, experiments are operated based on manual user input. Due to the complexity of operating biomedical imaging instruments and data analysis methods, setting up and even reproducing an experiment requires users to have background knowledge and experiences. Furthermore, reproduction is particularly difficult since not all the scientific publications follow a standardized form to described data acquisition and analysis methods. Instead, using synonyms, jargon, or vendor-specific terms introduces additionally challenges to reproduce the experiments. Hence, there may be a need for improved experiment automation and image analysis workflow to enhance reproducibility of experiments.

### Brief description of the Figures

Some examples of apparatuses and/or methods will be described in the following by way of example only, and with reference to the accompanying figures, in which
Fig. 1 shows a flowchart of an example of a method of generating an instrument setting;
Fig. 2 shows an example of a default schema;
Fig. 3 shows another example of a flowchart of the method of generating an instrument setting;
Fig. 4 shows a flowchart of example of a method of training a language model for a medical instrument;
Fig. 5 shows a schematic illustration of a system for performing biomedical imaging experiments;
Fig. 6 shows a schematic illustration of a system for running biomedical imaging experiments.

### Detailed Description

Some examples are now described in more detail with reference to the enclosed figures. However, other possible examples are not limited to the features of these embodiments described in detail. Other examples may include modifications of the features as well as equivalents and alternatives to the features. Furthermore, the terminology used herein to describe certain examples should not be restrictive of further possible examples.

Throughout the description of the figures same or similar reference numerals refer to same or similar elements and/or features, which may be identical or implemented in a modified form while providing the same or a similar function. The thickness of lines, layers and/or areas in the figures may also be exaggerated for clarification.

When two elements A and B are combined using an "or", this is to be understood as disclosing all possible combinations, i.e. only A, only B as well as A and B, unless expressly defined otherwise in the individual case. As an alternative wording for the same combinations, "at least one of A and B" or "A and/or B" may be used. This applies equivalently to combinations of more than two elements.

If a singular form, such as "a", "an" and "the" is used and the use of only a single element is not defined as mandatory either explicitly or implicitly, further examples may also use several elements to implement the same function. If a function is described below as implemented using multiple elements, further examples may implement the same function using a single element or a single processing entity. It is further understood that the terms "include", "including", "comprise" and/or "comprising", when used, describe the presence of the specified features, integers, steps, operations, processes, elements, components and/or a group thereof, but do not exclude the presence or addition of one or more other features, integers, steps, operations, processes, elements, components and/or a group thereof.

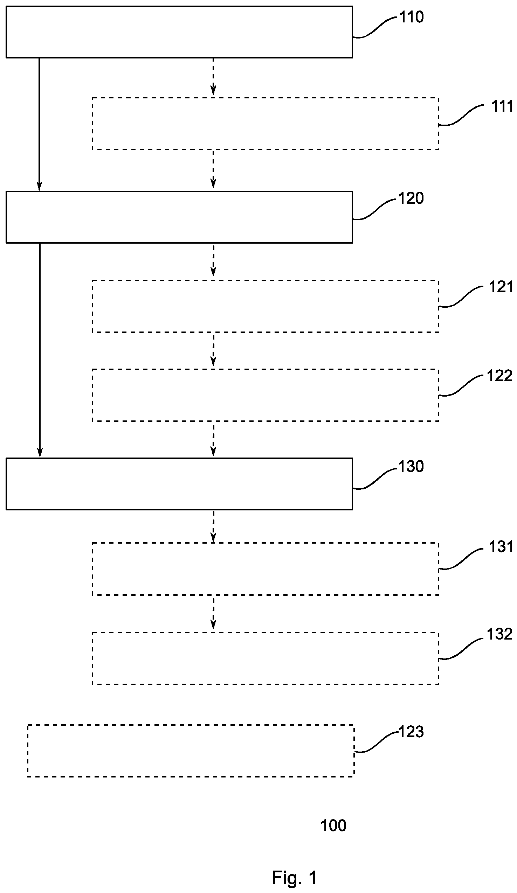

**Fig. 1** illustrates a flow chart of an example of a method 100 of generating an instrument setting. The method 100 comprises providing a scientific publication as an input 110, using a pre-trained language model on the input 120 to extract embeddings of keys and corresponding values associated to settings of a medical instrument, and generating the instrument setting 130 based on the embeddings and the corresponding values. The instrument setting causes the medical instrument to perform a task when applied to the instrument.

Optionally, the keys are descriptions of parameters of the medical instrument and the values represent the setting of the parameters of the medical instrument.

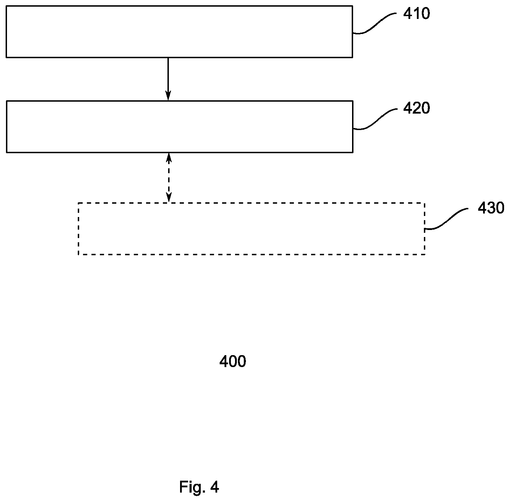

Embeddings in a large language model can be understood as representation of words, phrases, or sentences in a continuous vector space. The embeddings capture the semantic meaning and contextual relationships between words. In general, each word or token in the input text is converted into a high-dimensional vector, where similar words are represented by vectors that are closer together in the vector space. The embeddings are learned during a training process of the language model where the model adjusts the vectors to minimize the difference between similar words and maximize the difference between dissimilar words based on the context they appear in. This process enables the model to understand the meaning of words and their relationships within sentences, allowing it to generate coherent and contextually relevant responses. As described above, the proposed method 100 is based on the pre-trained language model for learning semantics of biomedical terminologies, instrument parameters and associated values, and information on specimen, sample preparation. The training of the language model is described in **Fig. 4****.**

For example, the pre-trained language model is optionally a large language model, advantageously encoder-decoder type with a transformer architecture. In this context, the encoder-decoder type can be understood as a framework designed to handle various types of input-output mapping. The encoder processes the input data and converts it into a fixed-sized representation or context vector. The decoder takes the context vector produced by the encoder and generates the output sequence from it. The transformer architecture follows the encoder-decoder type by handling sequential data, like text to draw global dependencies between the input and output.

Modern biomedical instruments may require a broad background knowledge in multiple domains to operate the instruments and associated systems and to analyze data acquired using the instruments. For example, imaging using a microscope requires understanding principles of microscopy, such as magnification, resolution, contrast, light sources, types of specimens, sample preparation, and data processing after acquisition. Therefore, setting up all parameters to perform an experiment using the microscope properly might be a time-consuming process, and there are possibilities of involving human errors, even for experts in the domain, in operating the instruments as well.

In scientific research, experimental results are communicated through journal articles and conference contributions. These results need to be reproducible by different research groups based on the methods used. Currently, automation systems in research fields streamline and optimize various aspects of data acquisition, analysis, and control processes. They leverage artificial intelligence (AI) and robotics to enhance efficiency, accuracy, and reproducibility in experiments by reducing the need for manual intervention and minimizing human error. For example, automation in microscopy enables high-throughput screening, real-time monitoring, and precise control of experimental conditions, leading to accelerated research discoveries, improved diagnostic workflows, and enhanced productivity in scientific and clinical settings.

However, validating the reproducibility of published results is typically carried out manually, relying on free-text descriptions of the methods. Unfortunately, these descriptions often lack standardized formats, leading to the use of synonyms, jargon, or vendor-specific terminology to describe experimental procedures. Jargon can be understood as a specialized terminology understood within a particular profession or group but may be challenging for those outside the field to comprehend. Understanding the entire acquisition workflow and data processing steps may necessitate an involvement of experienced experts in the relevant domains to reproduce the experiments accordingly. Therefore, there may be a demand for automated methods or systems capable of generating instrument settings to reproduce experiments based on scientific publications.

The method 100 may facilitate reproducing experiments using published scientific documentations by providing a scientific publication as an input 110 and using the pre-trained language model 120 to extract embeddings of keys and corresponding values associated to settings of a medical instrument 120, without intervention of a user to understand the methodology in the provided scientific publication.

While extracting embeddings of keys and values, the key-value pairs may still contain synonyms or meronyms, which needed to be resolved. Entity resolution in the context of a language model can be understood as a process of identifying, linking, and disambiguating keys across different parts of a text or across different texts to ensure consistency and accuracy in understanding and generating language.

Creating word embeddings involves mapping words or phrases to vectors of real numbers, embeddings. These embeddings capture the semantic meanings, relationships, and various linguistic features of words. In this context, entity resolution can be understood as a process to resolve the problem by mapping many possible synonyms to one key in a semantically correct way based on the vectors.

The latent space can be understood as an abstract, high-dimensional space where the pre-trained language model learns to encode patterns from the input data in the training process. The semantics are captured by creating vectors of embeddings of the keys in the latent space. The resulting embeddings for, e.g. synonyms are compared using a suitable metric, as an example, such as cosine similarity to measure the angle between the vectors. Those, which are closest, are matched with the respective terms in the data schema and the key-value pair from the corrected set of lists are used in the final instrument setting for generating an instrument setting 130.

There are different practical possibilities to perform entity resolution. One example is using sparse and long vectors of size |*V*|, which is a length of the vocabulary of a training data set of the pre-trained language model. It can utilize embeddings based on a term-frequency-inverse-document-frequency (TF-IDF). TF-IDF is numerical statistic that reflects how important a word is to a document in a collection or corpus. It is often used in text mining and information retrieval to evaluate the relevance of a term to a document in the context of a corpus. how frequently a term occurs in a document. Inverse Document Frequency measures how important a term is. While computing TF, all terms are considered equally important. However, certain terms, such as "is", "of", and "that", may appear a lot of times but have little importance. Thus, those frequent terms are weighted down while the rare ones are scaled up. In this example, each word is represented as a row in a term-document matrix of size |*V*| times D, wherein D is the number of input documents.

Alternatively, positive pointwise mutual information (PPMI) can be used. PPMI can be understood as a statistical used in natural language processing to quantify an association between two words, going beyond simple co-occurrence frequency. In PPMI, interactions (context) of each word are represented in a sparse term-term matrix of size |*V*| times |*V*| in this example. PPMI is widely used in natural language processing, especially in tasks involving semantic similarity and word embeddings for synonym detection.

Another example is to use short and dense vectors of arbitrary size *d* « |*V*|, where d may, for example, be smaller than 300, smaller than 400, smaller than 500, smaller than 700 or smaller than 1000. It uses static embeddings based on, as an example, training a skip-gram classifier with negative sampling. The skip-gram aims to predict the context words for a given target world, thereby learning representations that reflect linguistic patterns and semantic similarities. The negative sampling is an optimization to make this training process more efficient and scalable. Secondly, dynamic embeddings can be used, such as, as an example, a bidirectional encoder representation from transformers (BERT) transformer with a masked language model target. BERT can be understood as a method in natural language processing, which designed to understand the context of a word based on all of its surroundings. In other words, left and right of the word. Prior models typically read text unidirectionally (either left-to-right or right-to-left), which can limit understanding. BERT, by contrast, uses the Transformer's attention mechanism to consider the full context of a word by looking at the words that come before and after it, leading to a richer representation of text.

Lastly, entity resolution can be performed by retrieval augmented generation (RAG). An alternative to resolving entities in latent space is to use database lookup. To this end special tokens are inserted into the prompt to the pre-trained language model which are picked up by an orchestration layer built into the application. This will use the entity in question and perform a query of available databases, such as Wikipedia, Wikidata or a domain-specific knowledge database describing entities specific to the imaging device, lab automation system or image analysis software. Preferably, Wikidata can be advantageous over Wikipedia since it is structured for automated retrieval and has shorter descriptions and synonyms which can be looked up.

The method 100 optionally further comprises using a default schema 111. The default schema comprises at least a complete set of keys and associated values of an instrument setting required to make the medical instrument perform a task. The default schema would so enable to make the instrument perform a task without the user input.

The choice of method often depends on the specific requirements of the application, the availability of labeled data, and the computational resources at hand.

The method 100 optionally further comprises deriving missing embeddings 121 of keys and values that are not extracted from the user input but required for the instrument setting, wherein the missing keys and values are derived using the default schema. There is a possibility that some parameters are not extracted in step 120. Not all scientific publications have comprehensive description of all technical parameters of medical instruments and lab automation system. It could be due to involuntary omissions, but also, because users of such devices may not have full visibility of all parts of the device or workflow used, some parts of the device may have been configured automatically or the user is lacking the technical expertise to be aware of all parts. In this case, the pre-trained language model may not be able to simply guess missing keys and values, as setting hardware parameters wrongly might lead to damage of the instrument or harm to the operating user.

For example, while searching for unknown keys, or tokens, in the input, a special token, such as *UNK,* can be employed to maintain word size under control. These words comprising the *UNK* token may be possible candidates for missing values. The encoder part of the pre-trained language model generates a latent vector from the unknown tokens, which is then compared with latent vectors created from all the other tokens, e.g. keys, in the input. A detailed explanation of the method used to identify missing keys and values will be provided subsequently.

An exemplary method to derive the missing keys and values is to extract them from the default data schema by taking means between upper and lower bounds of respective parameter ranges. Alternatively, as an example, p-th percentile between upper and lower bound of the missing keys in the default schema can be used to derive missing keys and values.

The method 100 optionally further comprises optimizing keys and values iteratively 122 by comparing the extracted embeddings with the imputed key-value pairs and embeddings of published data using forward inferencing. Even though missing keys and values are derived from the default schema, it may not be the optimal setting depending on samples and instruments used. Forward inferencing can be understood as a reasoning process used in AI, where the reasoning process starts with a known situation to infer a new situation. In the context of optimizing keys and values, forward inferencing starts from the starting points derived from the default schema to infer optimized key-value pairs.

For example, quantized variational autoencoders (qVAE) can be used to optimize the imputed key-value pairs. VAE can be understood as a generative model used in unsupervised learning. VAE is designed to learn a latent representation of input data, which can then be used to generate new data points that are similar to the training data. In VAE, the latent space is typically continuous, which indicates that each latent variable can take on any value within a certain range. However, in qVAE, the latent space is discretized into a finite number of values. This discretization can be understood as dividing the continuous latent space into a grid of discrete point. The discretization step to the training process may help improved interpretability, stability, or computational efficiency. Using a compressed latent space of a trained qVAE, an image acquired using the extracted embeddings of keys and values and the imputed key-value pairs is compared to the image of the input data. The comparison is performed iteratively using a numerical metric for how closely the output matches the published data after doing forward inference of both to obtain vector embeddings of published and reproduced data, respectively. As an example, the metrics may involve L1 norm, L2 norm, p-norm, or cosine similarity.

As described above, generating the instrument setting 130 based on the embeddings and the corresponding values, wherein the instrument setting causing the medical instrument to perform a task.

The method 100 optionally further comprises enforcing integrity constraints of the medical instrument 131 by adapting the values corresponding to one or more keys. As described above in optimizing keys and values iteratively 122, wrong parameter setting can cause damage to the instrument hardware or even the operator of the instrument. Therefore, for safety reasons, enforcing integrity constraints of the medical instrument 131 may need to be followed by generating the instrument setting 130.

The method 100 optionally further comprises using the pre-trained language model to extract further embeddings of keys and corresponding values associated to a workflow of a data analysis system. The publication provided as the input comprises data analysis procedure together with instrument setting to acquire the data. It is possible to extract embeddings of keys and corresponding values associated to a workflow of a data analysis system, which may further reduce human error in experiment.

As an example, missing embeddings of the keys and values can also be derived from the default schema. For optimization of the imputed key-value pairs for the workflow of a data analysis system, an intermediate activation of a convolutional neural network (CNN), such as in perceptual loss, Pixel-based metrics, such as mean squared error, mean absolute error or structural similarity index or PSNR might be used. CNN is widely used pattern recognition in grid-like data, such as images.

The method 100 optionally 100 further comprises using the instrument setting to operate a microscope 132, which enables automated instrument operation for reproducing experiment. It can reduce manual instrument setting time and human error.

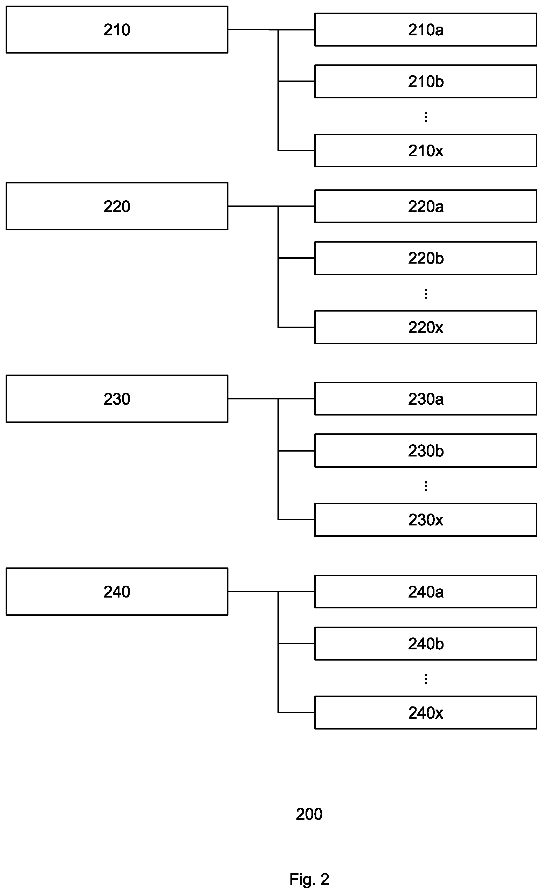

**Fig. 2** illustrates, amongst others, an exemplary default schema 210 for the instrument. Fig. 2 schematically illustrates an exemplary schematic diagram 200 of schemas comprising not only keys and values for the instrument (instrument schema 210) but also for other aspects eventually required to redo an experiment. As an example for such a setting, diagram 200 illustrates an instrument schema 210, an analysis schema 220, an experiment schema 230, and a user schema 240. As an example, the instrument schema 210 may comprise names of parameters, their lower and upper bounds, and units if applicable, such as {"LED 488" : {"type" : int, "lower" : 0, "upper" : 100, "unit" : "%"} 210a, {"storage-x" : {"type" : float32, "lower" : 0, "upper" : 65983.7, "unit" : "µm"} 210b, ... , {"HyD" : {...} 210x}. The analysis schema 220 may comprise parameters related to the analysis of images, such as for example {"Detection" : {...} 220a, "Background" : {"type" : int, ...} 220b, ... , "Contrast threshold" : {...} 220x}. The experiment schema 230 may comprise information on species, model organism, model system (e.g. cell culture, organoid, 3D cell culture, tissue graft, tissue section, and organ), staining and clearing procedures (immunofluorescence, tagged fluorescent proteins, low molecular weight protein tags for fluorescence, HE staining). Optionally, the user schema 240 may define further customized files by providing a free-text description, such as {"summary" : "Provide a concise summary of the methods part", "imaging workflow" : "Return a graph of all microscopic imaging steps as a dictionary with keys corresponding to known instrument parameters and values as in the test"}. It is worth noting that this is an exemplary non exhaustive list of parameter names and associated values with upper and lower bounds and units, which can be used in one particular microscopy experiment.

Optionally, prompting can be partially automated by using RAG as described above. A default schema and the user schema may be provided as nested dictionaries. Concatenations of such keys, e.g. {"Photomultiplier" : {"type" : int, "lower" : 0, "upper" : 900, "gain" : 700, "unit" : "volt"}} is concatenated as "photomultiplier volt", may be suitable for querying a database to retrieve a description which can be used as one element of reasoning. It is worth noting that it is not apparent known which combinations result in a reasonable sense. Therefore, all possible key combinations can be generated as search terms. Optionally, using a priori knowledge, some keys can be omitted in order to reduce search time.

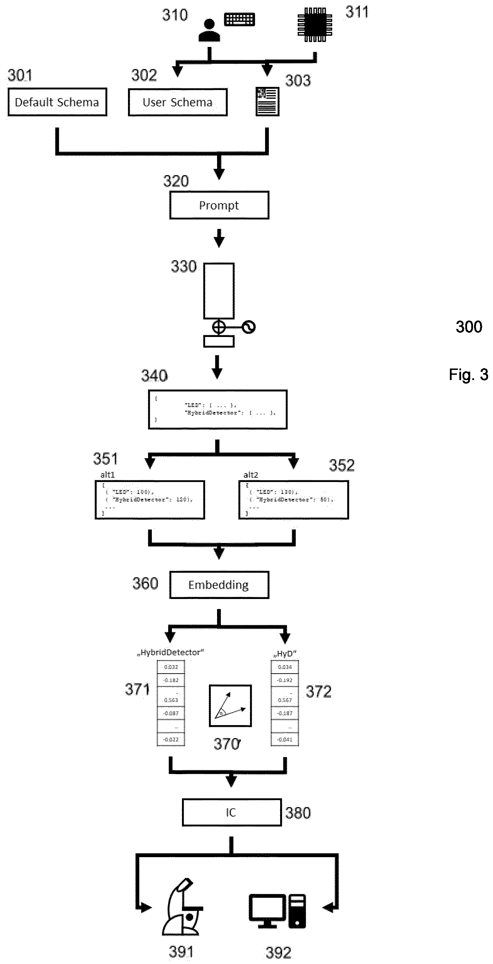

**Fig. 3** again illustrates an exemplary flowchart 300 of a method to generate an instrument setting. In this context, instrument settings are generated for a microscope 391 and a data analysis system 392.

A default schema 301 defines fields and data types for each field corresponding to instrument parameters, image analysis parameters, experiment parameters, and optionally a user schema can be provided 302 similar to Fig. 2.

A user input 310 is providing a publication of 303 which is to be reproduced. The publication 311 can be found by a literature search, using experiment metadata or image features. The metadata, in this context, is keys and associated values used to acquire an image.

A pre-trained language model 330 is used or prompted 320 in order to extract key-value pairs as information and output it as a nested dictionary 340 as described above in Fig. 1. These key-value pairs which were extracted are placed on a graph. In this context, the graph can be understood as a data structure used to represent a set of objects, nodes, along with the relationships or connections between these objects. Each node corresponds to the extracted key-value pair. In the simplest case the graph is a linear list, which is a graph of depth 1. Therefore, a unique list of key-value pairs is extracted. However, in other cases there may be alternatives at multiple steps, which is graphs of depth > 1. For example, the publication of interest 303 may comprises multiple image acquisition parameter values for the same parameter or multiple data analysis parameter values for the same parameter for comparison or referring to references. In such cases, a graph may need to be represented as an adjacency list and a set of lists can be created representing different extracted key value pairs 351 and 352.

There can be several data issues comprising missing keys and values, usage of synonyms, jargons, or vendor-specific terminologies, and duplicates, which needs to be solved before generating the instrument setting or image analysis workflow. The entity resolution and imputing missing keys may be performed as described above. As an example, synonyms, "HybridDetector" 371 and "HyD" 372, may comprised in the key-value pairs, which needs to be solved. In the entity resolution, the semantics are captured by creating a vector embedding of the keys 360 compared using a suitable similarity metric 370. In Fig. 3, as an example, a cosine similarity is presented. The cosine metric calculates the cosine of the angle between two vectors of the embeddings which reflects how closely related the two embeddings are in terms of their orientation, regardless of their magnitude.

Before applying the instrument setting or data analysis setting to the microscope 391 or the data analysis system 392, enforcing integrity constraints 380 is performed in order to avoid damage of the systems 391 and 392 or harm to an operating user. Finally, the settings can be applied to the imaging device 391, lab automation system or the data analysis system 392.

As an example, in Fig 3, prompting 320 or using the pre-trained language model 330 with the user input 303 can be described as, "Find experimental conditions related to microscopy and output a dictionary of key-value pairs. Values may be nested dictionaries.". Just as a very simple example, one may assume that a user input comprises the following description " The gain on the photomultiplier tubes (PMTs) was set to 700 V. To achieve a good time-resolution the highest scan speed available was used. Resonant scanning mode was used at 12000 Hz line frequency and Zoom 6 with 63x HCS Plan APO objective.". After following steps as described above (e.g. 340 - 380 in Fig. 3), the final instrument setting can be "Output: "PMT" : {"gain" : 700}, "Resonant scanner" : {"speed" : 12000, "zoom" : 6}, "objective" : {"magnification : 63x, "planarity" : "Plan APO"}}". This structured instrument setting can be sent to the microscope 391 or any medical instrument to reproduce an experiment described in the user input 303.

**Fig. 4** illustrates a flow chart of a method 400 of training a language model for a medical instrument. The method 400 comprises generating a training data set 410 from technical documentations of the medical instrument and training a language-based model 420 using the training data set, configured to generate embeddings of keys and associated values, the keys being descriptions of parameters of the medical instrument and the values representing the setting of the parameters of the medical instrument.

As an example, referring to Fig. 1, the keys are biology or medicine-related terminologies, descriptions of parameters of experiment instruments, lab automation systems, and data analysis systems or parts of experiment instruments, lab automation systems, and data analysis systems comprising illumination sources, photo detectors, scanning devices, spectroscopy instruments, actuator, and motors.

As an example, the language-based model is optionally a medium-sized language model. A language model is a computational model that is trained to predict the probability of a sequence of words or tokens in a language. Large language model comprises hundreds of billions or even trillions of parameters to be trained on extensive datasets, which requires substantial computational power for both training and inference. The medium-sized data model comprises significantly fewer parameters, ranging from tens of millions to tens of billions. It is more manageable in terms of computational requirements while providing a wide range of natural langue processing tasks effectively.

As an example, the medium-sized language model is optionally fine-tuned on prompting, comprising a chain-of-thought strategy, to improve prediction performance.

Fine-tuning a medium-sized language model involves adjusting parameters of the pre-trained model slightly to specialize it for a specific task or domain. The chain-of-thought strategy can be understood as a technique used in natural language processing to solve complex problems by breaking them down into intermediate steps or reasoning paths. In the context of language models, chain-of-thought prompting involves providing the model with a prompt that not only asks a question but also suggests a method for approaching the problem, often by outlining a series of logical steps or considerations. The model then generates text that follows this suggested path of reasoning, ideally leading to a more accurate and explainable conclusion.

There are several advantages of fine-tuning of the medium-sized language model using chain-of-thought prompting. The model generates intermediate steps or reasoning that leads to the final answer, making the problem-solving process more transparent and understandable. Also, by breaking down complex problems into simpler components, the model can more effectively tackle tasks that require multi-step reasoning. A user can craft prompts that guide the model in a specific direction, tailoring the reasoning process to fit the problem at hand.

For example, a prepended instruction is given to the medium-sized language model, such as "Find experimental conditions related to microscopy and output a list of key-value pairs.". Then, two exemplary sub-prompts comprising the chain-of-thought-style reasoning can be as follows:
Method: The gain on the photomultiplier tubes (PMTs) was set to 700 V. Output: [("PMT", 700)]. Reasoning: A photomultiplier, abbreviated 'PMT' is an electrical photosensor which has an amplification gain. The gain is measured in volts, abbreviated 'V'. Hence the key is PMT and the value 700.
Method: To achieve a good time-resolution the highest scan speed available was used. Resonant scanning mode was used at 12000 Hz line frequency and Zoom 6. Output: [("Resonant scanner", 12000), ("Zoom", 6)]. Reasoning: A resonant scanner is one particular realization of a scanner. A scanner is a device for moving a light beam across a sample to take measurements. The movement speed is measure as a frequency, abbreviated 'Hz'. Depending on the amplitude of the scanner a zoom is realized with a dimensionless numerical factor relative to the amplitude with no zoom.

In the fine-tuning step, based on the prepended instruction, keys, e.g. PMT, Resonant Scanner, Zoom, and their associated values, e.g. 700, 12000, 6, are extracted as outputs and reasonings in the connection with the chain-of-thought as described above are presented. The chain-of-thought strategy mirrors human cognitive processes, where complex problems are often solved by breaking them down into simpler components and reasoning through them sequentially. Moreover, by evaluating each step in the reasoning process, it helps in minimizing the propagation of errors.

As an example, the method 400 optionally further comprises querying a database to retrieve a description as one element of reasoning in the CoT strategy. It is possible that the training data set may not comprise all terminology used in scientific experiments and studies. In such a case, a database, such as such as Wikipedia, Wikidata or a domain-specific knowledge database describing entities specific to the imaging device, lab automation system or image analysis software can be retrieved to improve reasoning in the CoT strategy.

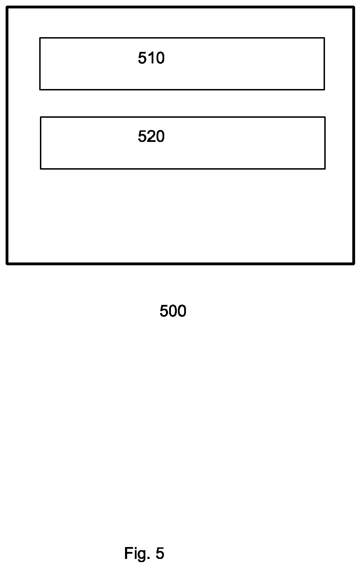

Fig. 5 illustrates a schematic diagram of an example of a system 500 for performing biomedical imaging experiments using published scientific articles, lab notes, or technical documentations, the system comprising one or more processors 510 and one or more storage devices 520. The system 500 may be used to perform various tasks. For example, the system 500 may be configured to perform the method 100 shown in connection with Fig. 1. Alternatively, or additionally, the system 500 may be configured to perform the method 400 shown in connection with Fig. 4.

More details and aspects of the system 500 are mentioned in connection with the proposed concept or one or more examples described above (e.g., Figs. 1 to 4). The system 500 for performing biomedical imaging experiments comprise one or more additional optional features corresponding to one or more aspects of the proposed concept, or one or more examples described above.

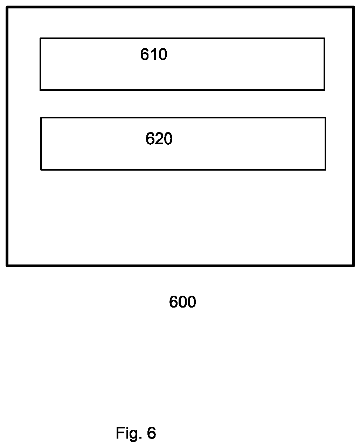

Fig. 6 illustrates a schematic diagram of an example of a system 600 for running biomedical imaging experiments, the system comprising one or more processors 610 and one or more storage devices 620. The system is configured to obtain experiment information in a form of scientific publications, lab notes, or technical documents to extract embeddings of key-value pairs from the obtained experiment information the method 100 shown in connection with Fig. 1 using a language-based model. The language-based model is trained according to the method 400 shown in connection with the method 400 shown in connection with Fig. 4. The system is configured to use the key-value pairs to operate biomedical imaging devices, analysis systems, or lab automation systems.

More details and aspects of the system 600 are mentioned in connection with the proposed concept or one or more examples described above (e.g., Figs. 1 to 4). The system 600 for performing biomedical imaging experiments comprise one or more additional optional features corresponding to one or more aspects of the proposed concept, or one or more examples described above.

Generally, embodiments of the present invention can be implemented as a computer program product with a program code, the program code being operative for performing one of the methods when the computer program product runs on a computer. The program code may, for example, be stored on a machine-readable carrier.

Other embodiments comprise the computer program for performing one of the methods described herein, stored on a machine-readable carrier.

In other words, an embodiment of the present invention is, therefore, a computer program having a program code for performing one of the methods described herein, when the computer program runs on a computer.

The examples described herein may be summarized as follows:
An example (e.g. example 1) relates to a method of generating an instrument setting, comprising: providing a scientific publication as an input; using a pre-trained language model on the input to extract embeddings of keys and corresponding values associated to settings of a medical instrument; and generating an instrument setting based on the embeddings and the corresponding values, the instrument setting causing the medical instrument to perform a task.

Another example (e.g. example 2) relates to a previously described example (e.g. example 1), wherein a respective impulse response function of the plurality of filters is fixed.

Another example (e.g. example 3) relates to a previously described example (e.g. example 1), further comprising: providing a default schema, the default schema comprising a complete set of keys and associated values of an instrument setting required to make the medical instrument perform a task.

Another example (e.g. example 3) relates to a previously described example (e.g. example 1 or 2), wherein the keys are descriptions of parameters of the medical instrument and the values represent the setting of the parameters of the medical instrument.

Another example (e.g. example 4) relates to a previously described example (e.g. example 1, 2, or 3) further comprising: further comprising: deriving missing embeddings of keys and values that are not extracted from the user input but required for the instrument setting.

Another example (e.g. example 5) relates to a previously described example (e.g. example 4), wherein the missing keys and values are derived using the default schema.

Another example (e.g. example 6) relates to a previously described example (e.g. example 4 or 5), further comprising: optimizing keys and values iteratively by comparing the extracted embeddings with imputed key-value pairs and embeddings of published data using forward inferencing.

Another example (e.g. example 7) relates to a previously described example (e.g. example 1), further comprising: enforcing integrity constraints of the medical instrument by adapting the values corresponding to one or more keys.

Another example (e.g. example 8) relates to previously described examples (e.g. examples 1 to 7), further comprising: using the pre-trained language model to extract further embeddings of keys and corresponding values associated to a workflow of a data analysis system.

Another example (e.g. example 9) relates to previously described examples (e.g. examples 1 to 8), further comprising: using the instrument setting (123) to operate a microscope.

Another example (e.g. example 10) relates to a method of training a language model for a medical instrument, comprising: generating a training data set from technical documentations of the medical instrument; and training a language-based model using the training data set, configured to generate embeddings of keys and associated values, the keys being descriptions of parameters of the medical instrument and the values representing the setting of the parameters of the medical instrument.

Another example (e.g. example 11) relates to a previously described example (e.g. example 10), wherein the keys are biology or medicine-related terminologies, descriptions of parameters of experiment instruments, lab automation systems, and data analysis systems or parts of experiment instruments, lab automation systems, and data analysis systems comprising illumination sources, photo detectors, scanning devices, spectroscopy instruments, actuator, and motors.

Another example (e.g. example 12) relates to previously described examples (e.g. example 10 and 11), wherein the language-based model is a medium-sized language model.

Another example (e.g. example 13) relates to previously described examples (e.g. example 10 and 12), wherein the medium-sized language model is fine-tuned on prompting, comprising a chain-of-thought strategy, to improve prediction performance.

Another example (e.g. example 14) relates to previously described examples (e.g. example 10 and 13), further comprising: querying a database to retrieve a description as one element of reasoning in the chain-of-thought strategy.

Another example (e.g. example 15) relates to a system for performing biomedical imaging experiments using published scientific articles, lab notes, or technical documentations, the system comprising one or more processors and one or more storage devices , wherein the system is configured to perform at least one of the method of one of the examples 1 to 9 and the method of one of the examples 10 to 14.

Another example (e.g. example 16) relates to a system for running biomedical imaging experiments, the system comprising one or more processors and one or more storage devices (6, wherein the system is configured to obtain experiment information in a form of scientific publications, lab notes, or technical documents, extract embeddings of key-value pairs from the obtained experiment information according to the method of one of the examples 1 to 8 using a language-based model that is trained according to the method of one of the examples 10 to 14; and use the key-value pairs to operate biomedical imaging devices, analysis systems, or lab automation systems.

Another example (e.g. example 17) relates to a computer program with a program code for performing the method according to one of the examples 1 to 14 when the computer program is run on a processor.

The aspects and features described in relation to a particular one of the previous examples may also be combined with one or more of the further examples to replace an identical or similar feature of that further example or to additionally introduce the features into the further example.

Examples may further be or relate to a (computer) program including a program code to execute one or more of the above methods when the program is executed on a computer, processor or other programmable hardware component. Thus, steps, operations or processes of different ones of the methods described above may also be executed by programmed computers, processors or other programmable hardware components. Examples may also cover program storage devices, such as digital data storage media, which are machine-, processor- or computer-readable and encode and/or contain machine-executable, processor-executable or computer-executable programs and instructions. Program storage devices may include or be digital storage devices, magnetic storage media such as magnetic disks and magnetic tapes, hard disk drives, or optically readable digital data storage media, for example. Other examples may also include computers, processors, control units, (field) programmable logic arrays ((F)PLAs), (field) programmable gate arrays ((F)PGAs), graphics processor units (GPU), application-specific integrated circuits (ASICs), integrated circuits (ICs) or system-on-a-chip (SoCs) systems programmed to execute the steps of the methods described above.

It is further understood that the disclosure of several steps, processes, operations or functions disclosed in the description or claims shall not be construed to imply that these operations are necessarily dependent on the order described, unless explicitly stated in the individual case or necessary for technical reasons. Therefore, the previous description does not limit the execution of several steps or functions to a certain order. Furthermore, in further examples, a single step, function, process or operation may include and/or be broken up into several sub-steps, - functions, -processes or -operations.

If some aspects have been described in relation to a device or system, these aspects should also be understood as a description of the corresponding method. For example, a block, device or functional aspect of the device or system may correspond to a feature, such as a method step, of the corresponding method. Accordingly, aspects described in relation to a method shall also be understood as a description of a corresponding block, a corresponding element, a property or a functional feature of a corresponding device or a corresponding system.

The following claims are hereby incorporated in the detailed description, wherein each claim may stand on its own as a separate example. It should also be noted that although in the claims a dependent claim refers to a particular combination with one or more other claims, other examples may also include a combination of the dependent claim with the subject matter of any other dependent or independent claim. Such combinations are hereby explicitly proposed, unless it is stated in the individual case that a particular combination is not intended. Furthermore, features of a claim should also be included for any other independent claim, even if that claim is not directly defined as dependent on that other independent claim.

## Claims

1. A method (100) of generating an instrument setting, comprising:
providing a scientific publication as an input (110);
using a pre-trained language model on the input (120) to extract embeddings of keys and corresponding values associated to settings of a medical instrument; and
generating an instrument setting (130) based on the embeddings and the corresponding values, the instrument setting causing the medical instrument to perform a task.

2. The method of claim 1, further comprising: providing a default schema (111), the default schema comprising a complete set of keys and associated values of an instrument setting required to make the medical instrument perform a task.

3. The method of claim 1 or 2, wherein the keys are descriptions of parameters of the medical instrument and the values represent the setting of the parameters of the medical instrument.

4. The method of any one of claims 1 to 3, further comprising:
deriving missing embeddings of keys and values (121) that are not extracted from the user input but required for the instrument setting.

5. The method of claim 4, wherein the missing keys and values are derived using the default schema.

6. The method of claim 4 or 5, further comprising: optimizing keys and values iteratively (122) by comparing the extracted embeddings with imputed key-value pairs and embeddings of published data using forward inferencing.

7. The method of claim 1, further comprising: enforcing integrity constraints of the medical instrument (131) by adapting the values corresponding to one or more keys.

8. The method of any claims 1 to 7, further comprising: using the pre-trained language model to extract further embeddings of keys and corresponding values associated to a workflow of a data analysis system (132).

9. The method of any one of claims 1 to 8, further comprising: using the instrument setting (123) to operate a microscope.

10. A method (400) of training a language model for a medical instrument, comprising:
generating a training data set (410) from technical documentations of the medical instrument; and
Training a language-based model using the training data set (420), configured to generate embeddings of keys and associated values, the keys being descriptions of parameters of the medical instrument and the values representing the setting of the parameters of the medical instrument.

11. A method of claim 10, wherein the keys are biology or medicine-related terminologies, descriptions of parameters of experiment instruments, lab automation systems, and data analysis systems or parts of experiment instruments, lab automation systems, and data analysis systems comprising illumination sources, photo detectors, scanning devices, spectroscopy instruments, actuator, and motors.

12. A method of any of claims 10 to 11, wherein the language-based model is a medium-sized language model.

13. A system (500) for performing biomedical imaging experiments using published scientific articles, lab notes, or technical documentations, the system comprising one or more processors (510) and one or more storage devices (520), wherein the system is configured to perform at least one of the method of one of the claims 1 to 9 and the method of one of the claims 10 to 14.

14. A system (600) for running biomedical imaging experiments, the system comprising one or more processors (610) and one or more storage devices (620), wherein the system is configured to
obtain experiment information in a form of scientific publications, lab notes, or technical documents;
extract embeddings of key-value pairs from the obtained experiment information according to the method of one of the claims 1 to 8 using a language-based model that is trained according to the method of one of the claims 9 to 12; and
use the key-value pairs to operate biomedical imaging devices, analysis systems, or lab automation systems.

15. A computer program with a program code for performing the method according to one of the claims 1 to 12 when the computer program is run on a processor.
